# EUROPEAN PATENT SPECIFICATION

(11) **EP 3 029 059 B1**
(45) Date of publication and mention of the grant of the patent: **04.07.2018**
(21) Application number: 14801111.7
(22) Date of filing: 21.05.2014
(51) Int. Cl.: C07H 21/02, C07H 21/04, C07K 17/06

(54) **COMPOUND ADMINISTRATION PRECURSOR AND MEDICAMENT CARRIER PREPARATION**
VERBINDUNGSVERABREICHUNGSVORLÄUFER UND ARZNEIMITTELTRÄGERHERSTELLUNG
PRÉCURSEUR D'ADMINISTRATION D'UN MÉDICAMENT ET FORMULATION D'UN VECTEUR DE MÉDICAMENT

(30) Priority: 21.05.2013 CN 201310190218
(43) Date of publication of application: 08.06.2016
(73) Proprietor: Hitgen Ltd., Sichuan 610041 (CN)
(72) Inventor: LI, Jin, Chengdu Sichuan 610041 (CN); YANG, Benyanzi, Chengdu Sichuan 610041 (CN); DOU, Dengfeng, Chengdu Sichuan 610041 (CN); GE, Xiaohu, Chengdu Sichuan 610041 (CN); SONG, Hongmei, Chengdu Sichuan 610041 (CN); WAN, Jinqiao, Chengdu Sichuan 610041 (CN); ZHANG, Yan, Chengdu Sichuan 610041 (CN); HU, Xiao, Chengdu Sichuan 610041 (CN); WANG, Xing, Chengdu Sichuan 610041 (CN); FENG, Jingchao, Chengdu Sichuan 610041 (CN); ZHONG, Guoqing, Chengdu Sichuan 610041 (CN)
(74) Representative: Inal, Aysegul Seda
(86) International application number: PCT/CN2014/077974
(87) International publication number: WO 2014/187315

(56) References cited:
- CN-A- 101 891 804
- CN-A- 101 899 092
- CN-A- 102 827 251
- US-A1- 2008 004 234

## Description

### TECHNICAL FIELD

The present invention relates to a compound drug-delivery precursor and a drug carrier preparation.

### BACKGROUND OF THE PRESENT INVENTION

The effectiveness and safety of drugs are closely related to pharmacokinetic characteristics in the human bodies (for example, delivery, expression of activity or toxicity and metabolism of drugs in human bodies), and these characteristics largely depend on the transmembrane transfer process of the drugs in the human bodies. Therefore, most scholars believe that the membrane permeability is one of important factors that will determine the safety and effectiveness of drugs. With wide application of combinational chemistry, genetic technology, high throughput screening technology in research and development of drugs, a large amount of active candidate drugs have been discovered. However, duo to a defect of low membrane permeability of drugs, many candidate drugs cannot enter cells to play a role as they are supposed to. Actually, many candidate drugs with low membrane permeability are strongly bioactive, and have excellent effects in fields of tumor, diabetes, cardiovascular diseases and the like. Therefore, how to improve the membrane permeability of drugs has become a hot and difficult issue of study of new drugs, and a new technology is urgently required to solve this problem. CN101899092 A (BEIJING UNIVERSITY, 1 December 2010) discloses a conjugate VIII having a peptide linked to RNA, wherein the peptide is membrane-permeable. US2008/004234 A1 (SEGEV DAVID [IL], 3 January 2008) discloses novel conjugates of oligomeric compounds to improve delivery of DNA/RNA molecules into living cells, wherein the oligonucleotides having low cell membrane permeability are biologically active. CN102827251A (THE FOURTH MILITARY MEDICAL UNIVERSITY, 19 Decembee 2012) discloses a transmembrane peptide-mediated antisense antibacterial agent. CN101891804A (THE FOURTH MILITARY MEDICAL UNIVERSITY, 24 November 2010) discloses a novel peptide-linker-oligonucleotide compound and a solid phase gradual synthesis method thereof. However, these documents do not disclose all of the features of the subject matter of the present invention.

### SUMMARY OF THE PRESENT INVENTION

An objective of the present invention is to provide a compound drug-delivery precursor for membrane permeation, and a drug carrier preparation based on this precursor.

The present invention provides a compound drug-delivery precursor, the structural formula of which is as follows:

X-linker-DNA/RNA Formula 1

where X refers to a compound with no membrane permeability, and linker refers to a linking group between X and DNA or RNA.

In the present invention, X refers to a compound with no membrane permeability. Such a compound is very likely to be abandoned during the research and development process since it cannot penetrate through the cell membrane during conventional use, or, cannot exhibit the best activity when in use. Such a compound is not limited to specific compounds used in embodiments of the present invention. Studies in the present invention show that the compound may have conditions for penetrating through the cell membrane after being linked to the DNA or RNA by the linker. Assisted by a gene transfer method, the membrane permeation and transfer of the compound X may be realized.

The "gene transfer" refers to a process of transferring nucleic acid into cells physically, chemically or biologically. The gene transfer method of the present invention refers to all transfer methods by which no obvious damage will be caused to cells. One of a well-known cationic liposome transfection method, a calcium phosphate transfection method, a nanoparticles transfection method or an electroporation transfection method and other technical methods capable of transferring nucleic acid into cells or a combination thereof may be used.

Further, the DNA or RNA is single-stranded or double-stranded.

Still further, the length of the single-strand or double-strand of the DNA or RNA is not less than five base pairs or bases.

Still further, the DNA or RNA is any sequence within a defined range of length. For example, single-stranded or double-stranded DNA or RNA has a length of 5 to 38 base pairs and bases.

In one specific implementation, the DNA or RNA may be: polyA of 5 bp, polyA of 19 bp, polyA of 38 bp, a single-stranded random sequence of 19 bp or a double-stranded random sequence of 19 bp. Further, the DNA or RNA has one functional group for covalent linkage.

Further, the molecular weight of the X ranges from 100 Da to 4000Da.

Still further, the X refers to a non-peptide compound or peptide compound with low membrane permeability.

Further, a linking site of the linker on the compound X has no influence on the bioactivity of the X.

Further, the linker is any covalent linkage enabling the compatible linkage and reaction between the compound X and the DNA or RNA, or may be a saturated and non-saturated covalent group capable of linking the compound to the DNA/RNA.

In the specific implementation, the linker is covalently linked to the compound X directly or by a pre-modified compound X.

In one implementation, the structural formula of the compound drug-delivery precursor prepared in the present invention may be as follows: 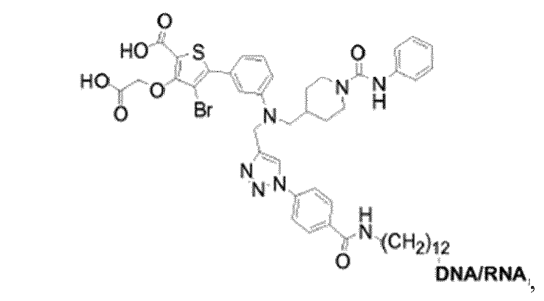 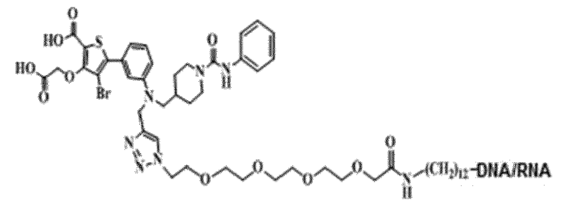 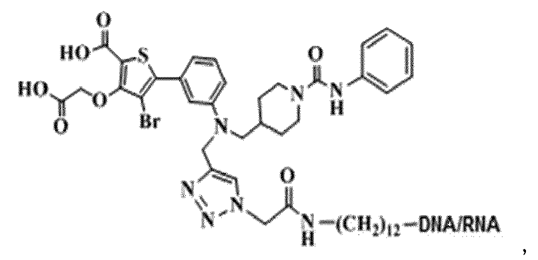 or

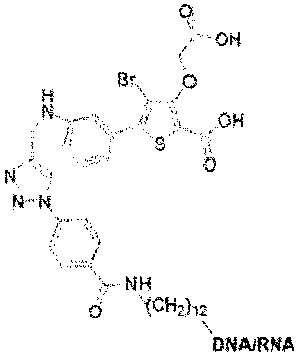

The present invention further provides a method for preparing the compound drug-delivery precursor, including the following operating steps of :
(1) Preparing raw materials: preparing a compound X and DNA or RNA with one reactive functional group;
(2) Preparing for covalent linkage of the compound X: selectively reacting the compound X with one functional group of a bifunctional reagent, to obtain a compound X1;
(3) Preparing for covalent linkage of the DNA or RNA: modifying the DNA or RNA with one reactive functional group with the bifunctional reagent, to obtain modified DNA or RNA matched with the covalent linkage of the compound X; and
(4) Covalently linking: covalently linking the compound X1 to the modified DNA or RNA matched with the compound X1, to obtain the compound drug-delivery precursor by separation and purification; or covalently linking the compound X1 to the DNA or RNA with one reactive functional group in the step (1), to obtain the compound drug-delivery precursor by separation and purification; or covalently linking the compound X to the modified DNA or RNA in the step (3), to obtain the compound drug-delivery precursor by separation and purification.

The "reactive functional group" refers to a group which is compatible with the DNA or RNA and capable of reacting with other reagents, for example, amino, sulphydryl, carboxyl, azido and the like; and the "bifunctional reagent" refers to a reagent containing two functional groups available for chemical reactions, for example, 4-azido-benzoic acid ester, which may have a reaction with amino prior to a click reaction with alkyne.

"Selectively reacting the compound X with one functional group of a bifunctional reagent" in the step (2) means that the linking site on the compound X has no influence on the bioactivity of the X after reaction with the compound X.

The present invention further provides a drug carrier preparation based on the above-mentioned compound drug-delivery precursor. The drug carrier preparation is prepared from the above-mentioned compound drug-delivery precursor and a carrier.

Further, the carrier is a biological material for transferring DNA or RNA. The biological material for transferring DNA or RNA is a transfection reagent for DNA or RNA. The "transfection" is a process where eukaryotic cells obtain new genetic markers due to the addition of exogenous DNA or RNA.

The present invention further provides a method for preparing the drug carrier preparation based on the above-mentioned compound drug-delivery precursor, including the following operating steps of:
(1) Preparing materials: weighing the compound drug-delivery precursor and the drug carrier in a proportion of 1:10-100 (W/V); and
(2) Incubating: uniformly mixing the compound drug-delivery precursor with a buffer solution to obtain a solution A; adding the drug carrier to the buffer solution and uniformly mixing to obtain a solution B; and mixing the solution A and the solution B, triturating by a pipette, and incubating at room temperature to obtain the drug carrier preparation.

The time for the incubation at room temperature may be simply selected according to the transmembrane effect. Any incubation time enough for the transmembrane transfer is applicable to the present invention. For example, in one embodiment of the present invention, the incubation at room temperature lasts for 20 min.

Taking the preparation of a drug carrier for transferring a compound drug-delivery precursor (25 nM) as an example, 0.125 µg of compound drug-delivery precursor was added to a sterile centrifuge tube (tube A) of 1.5 mL, and mixed uniformly with a buffer solution in a corresponding volume, with a total volume of 100 µL; 2.5 µL of transfer carrier was mixed with 97.5 µL of buffer solution in another tube (tube B), with a total volume of 100 µL; and the solution in the tube A was mixed with the solution in the tube B, and the mixture was slightly triturated by a pipette and incubated at room temperature to obtain a drug carrier for the compound drug-delivery precursor (25 nM).

The membrane permeability is the precondition for drugs to exert their pharmacological effects in the human bodies. The drug-delivery precursor and the drug carrier preparation of the present invention may effectively improve the membrane permeability of compounds with low membrane permeability, transfer drugs with no membrane permeability and low membrane permeability into cells, and provide a new choice for clinical medication.

In one specific implementation, the drug-delivery precursor and the drug carrier preparation of the present invention may be used to transfer drugs with low membrane permeability into cells, thus to exert or improve the pharmacological activity.

In another specific implementation, the drug-delivery precursor and the drug carrier preparation of the present invention may be used to transfer drugs with low membrane permeability into cells, so that the drugs are bound with target sites in the cells, thus to screen a binding site and a binding method of related drugs and target protein. This allows for targeted research on related drugs while sufficiently ensuring the integrity of cells, and meanwhile, this ensures the permeability has no influence on the research results of the related drugs and avoids eliminating the potential active ingredients.

Apparently, according to the content of the present invention, various modifications, replacements and alterations in other forms may be made in accordance with common technical knowledge and conventional methods of the art, without departing from the basic technical concept of the present invention.

The content of the present invention will be further described in detail by specific implementations in the form of embodiments. However, it should not be interpreted as limiting the scope of the subject of the present invention only to the following embodiments. Techniques realized based on the content of the present invention shall all fall into the scope of the present invention.

### BRIEF DESCRIPTION OF THE DRAWINGS

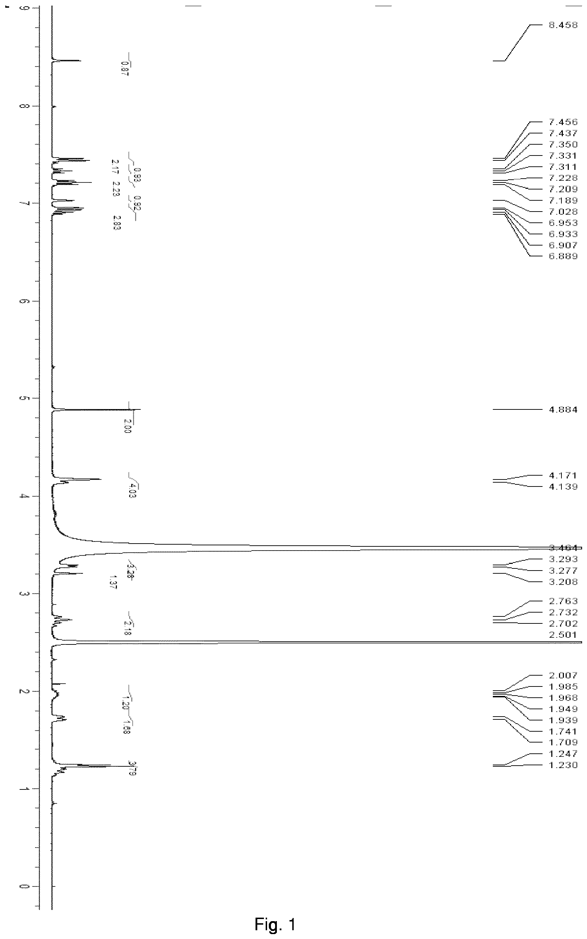

Fig. 1 is a¹H NMR curve of a compound 2;

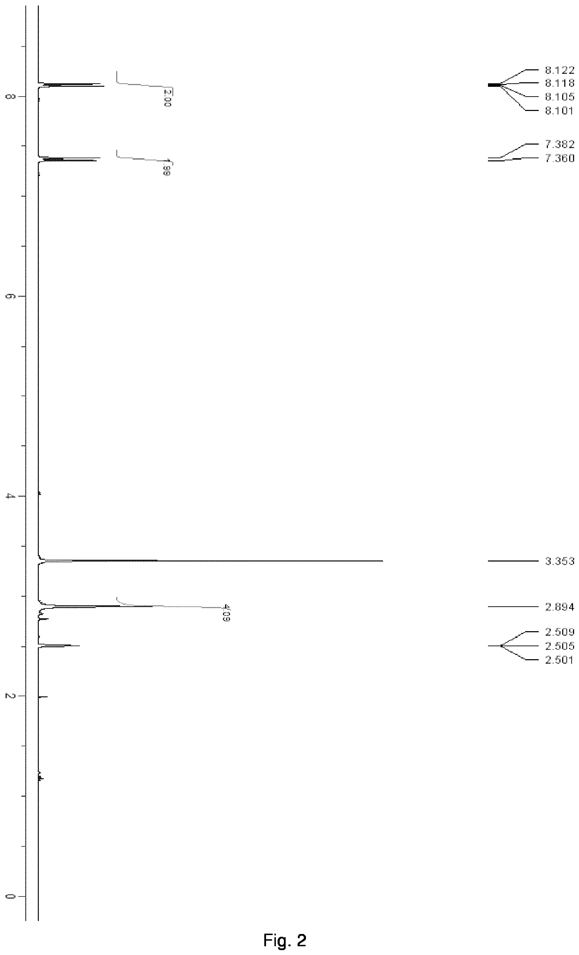

Fig. 2 is a¹H NMR curve of a compound 3;
Fig. 3 is a HPLC detection curve of a compound 5;
Fig. 4 is a mass spectrum of the compound 5;
Fig. 5 shows a PTP1 B inhibitor and IC50 measurement of the modified compound 2;
Fig. 6 shows measurement of the concentration of the compound 5;
Fig. 7 shows IC50 measurement of the PTP1 B by the compound 5;
Fig. 8 shows positioning, by laser confocal microscopy, in cell-penetrating experiments, of the compound 5 (those in blue are cell nucleus, and those in green are FITC-tagged compounds 5);
Fig. 9 shows the transfer efficiency of the compound 5; A) shows the total number of cells observed by a phase contrast microscope; B) shows the cells into which the drug-delivery precursor 1 is transferred, observed by a fluorescent microscope; and C) shows the transfer efficiency;
Fig. 10 shows the influence, on the phosphorylation of cells, of the drug-delivery precursor based on the PTP1B inhibitor and the drug carrier preparation; and
Fig. 11 shows positioning, by laser confocal microscopy, in cell-penetrating experiments, of drug-delivery precursors having single-stranded or double-stranded DNA or RNA of different length, different compounds and different linkers (those in blue are cell nucleus, and those in green are FITC-tagged single-stranded or double-stranded DNAs or RNAs).

### DETAILED DESCRIPTION OF THE PRESENT INVENTION

### Embodiment 1

### Synthesis of a compound drug-delivery precursor 1 of the present invention

### 1. Materials and reagents

Compound 1 was synthesized by our company according to the method as described in the reference document (D.P. Wilson et al, J. Med. Chem. 2007, 50, 4681-4698); polyA (5'-(CH2)12-A19-3'-FITC) modified by 5'-amino and 3'-fluorescein was manufactured by Invitrogen Trading Shanghai Co., Ltd; and the other reagents used for chemical synthesis were purchased from Aldrich or TCI.

### 2. Synthesis method

The drug-delivery precursor of the PTP1B inhibitor was synthesized according to the following route:

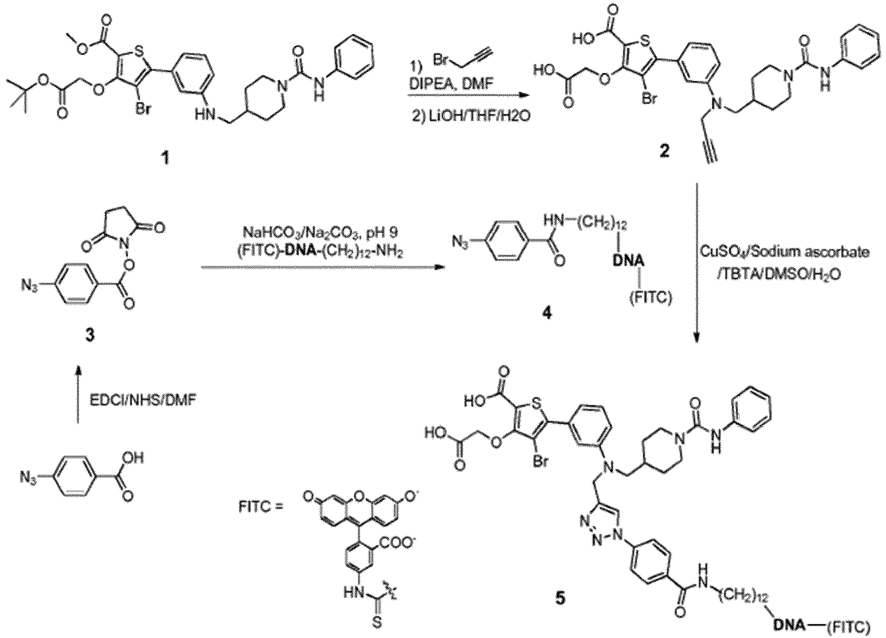

### Compound 2: 4-bromo-3-oxoacetic acid-5-(3-(((1-phenyl carbamoylpiperidine)-4-methyl)-N-propargyl amine) phenyl) thiophene-2-formic acid

4-bromo-3-oxo-tert-butyl acetate-5-(3-((1-phenyldiethyl carbamoylpiperidine)-4-methyl)phenyl) thiophene-2-methyl formate (1) (250 mg, 0.4 mmol), propargyl bromide (70 mg, 0.5 mmol) and N,N-diisopropylethylamine (1.5 mL) were dissolved into 20mL of N,N-dimethylformamide, stirred for 5h at 90°C, cooled to room temperature, and distilled under reduced pressure to obtain a crude product; and the crude product was separated by column chromatography to obtain 4-bromo-3-oxo-tert-butyl acetate-5-(3-(((1-phenyl carbamoylpiperidine)-4-methyl)-N-propargyl amine) phenyl) thiophene-2-methyl formate (white solid, 130 mg, with a yield of 49%). MS m/z (ESI): 668,670(M+H)⁺; 690,692 (M+Na)⁺Lithium hydroxide (200 mg, 2.38 mmol) was added to 4-bromo-3-oxo-tert-butyl acetate-5-(3-(((1-phenyl carbamoylpiperidine)-4-methyl)-N-propargyl amine) phenyl) thiophene-2-methyl formate (100 mg, 0.15 mmol) in 5 mL of tetrahydrofuran and 5 mL of aqueous solution, and stirred overnight at room temperature. 2N hydrochloric acid was added to the reaction solution; and the reaction solution was acidified until the pH became 2, and then concentrated to obtain a crude product. The crude product was treated by HPLC to obtain 4-bromo-3-oxoacetic acid-5-(3-(((1-phenyl carbamoylpiperidine)-4-methyl)-N-propargyl amine) phenyl) thiophene-2-formic acid (compound 1-3) (white solid, 40 mg, with a yield of 42%).
MS m/z (ESI): 626,628 (M+H)⁺;¹H NMR (CDCl3): δ8.45(s, 1 H), 7.43(m, 2H), 7.33(t,J= 7.6 Hz,1 H), 7.21 (m,2H), 7.03(s, 1 H), 6.92(m, 3H), 4.88(s, 2H), 4.15(m, 4H), 3.28(m, 2H), 3.20(m, 1H), 2.70(m,2H), 1.82(m,1H), 1.73(m,2H), 1.24(m,3H).

### Compound 3: 4-azidobenzoate succinimide ester

In ice bath, 1-ethyl-3-(3-dimethylamine propyl) carbodiimide hydrochloride (EDCI, 570 mg, 3.7 mmol) was added to 10 mL of N,N-dimethylformamide containing 4-azidobenzoic acid (500 mg, 3.06 mmol), and then N-hydroxysuccinimide (440 mg, 3.7 mmol) was added thereto. The reaction lasted for 1h away from light and under protection of nitrogen; and then, the reaction solution was heated to room temperature, and stirred overnight away from light. N,N-dimethylformamide was removed by distillation under reduced pressure; and then, the residues were dissolved in ethyl acetate and washed with water for three times; and finally, the organic phase was dried by anhydrous sodium sulfate, filtered and concentrated to obtain a crude product. The crude product was separated by column chromatography to obtain the product 4-azidobenzoate succinimide ester (5) (white solid, 780 mg, with a yield of 97.5%).¹H NMR (DMSO-d6): δ8.11(d,J= 8.4 Hz, 2H), 7.37(d,J= 8.4 Hz, 2H), 7.37(s, 4H).

### Compound 4: 4-azidobenzamide 12-alkyl 19 polyA fluorescein

A mixture of polyA (5'-(CH2)12-A19-3'-FITC) (50 nmol) modified by 5'-amino and 3'-fluorescein, the 4-azidobenzoate succinimide ester (3) (5 µmol,100 eq.)in 500 µL of 0.5 M sodium carbonate/sodium bicarbonate buffer solution (pH 9), and 500µL of dimethylsulfoxide was shaken overnight in a low speed at room temperature. Then, the reaction system was directly separated by reverse HPLC column chromatography, and lyophilized to obtain 4-azidobenzamide 12-alkyl 19 polyA fluorescein (4) (light yellow solid, with a yield of over 90%).

### Compound 5: 4-bromo-3-oxoacetic acid-5-(3-(((1-(4-fluorescein 19 polyA) 12-alkyl acetamidophenyl)-1H-1,2,3-triazole-4-methylene)(1-phenylcarbamoylpipe ridine)-4-methyl)amino)phenyl)thiophene-2-formic acid (i.e., the drug-delivery precursor 1 of the present invention)

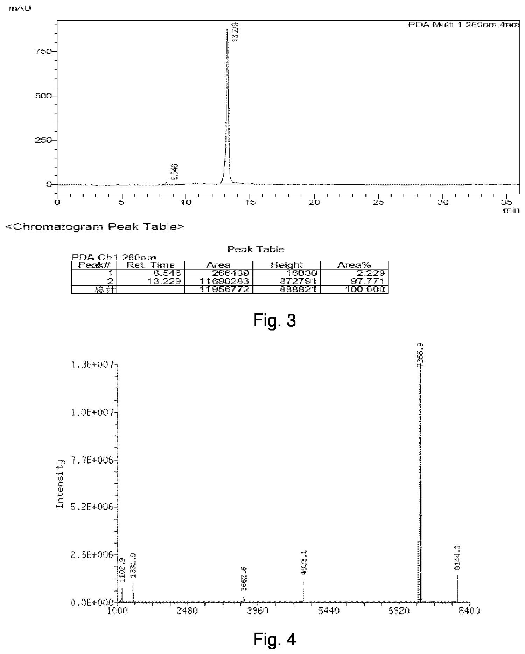

30 µL of solution A (copper sulfate and tris[(1-benzyl-1H-1,2,3-triazol-4-yl)methyl]amine were dissolved at a mole ratio of 1:2 into a solution composed of water, dimethylsulfoxide and tert-butanol at a volume ratio of 4:3:1, with a concentration of 10 mM) was added to solution B (4-azidobenzamide 12-alkyl 19 polyA fluorescein (4) (15 nmol) in 200 µL of aqueous solution and 4-bromo-3-oxoacetic acid-5-(3-(((1-phenyl carbamoylpiperidine)-4-methyl)-N-propargyl amine) phenyl) thiophene-2-formic acid (2) (960 nmol)) in 50 µL of DMSO solution, and vortex-centrifuged; and subsequently, 60 µL of newly-prepared sodium ascorbate (600 nmol) in aqueous solution was added to the reaction system, and then shaken overnight in a low speed at room temperature. Then, the reaction solution was directly separated by reverse HPLC column chromatography and purified to obtain the product 4-bromo-3-oxoacetic acid-5-(3-(((1-(4-fluorescein 19 polyA) 12-alkyl acetamidophenyl)-1H-1,2,3-triazole-4-methylene)(1-phenylcarbamoylpiperidin e)-4-methyl)amino)phenyl) thiophene-2-formic acid (5) (light yellow solid, with a yield of 80% and a concentration of 90%, see HPLC curve). Please refer to Fig. 4 for the mass spectrum.

FITC is a fluorescent tag. The purpose of adding the FITC in the present invention is merely for ease of observation of the compound drug-delivery precursor in experiments. The tag FITC is not an indispensable structure of the compound drug-delivery precursor of the present invention, similarly hereinafter.

### Embodiment 2

### Tests on the inhibition of the PTP1B directly by compound 2 and compound drug-delivery precursor 1 (compound 5)

### 1. Materials and reagents

The human full-length PTP1 B protein was purchased from Sigma (Cat# SRP0215, Lot# 3000920322); the substrate (4-nitrophenyl phosphate disodium salt (hexahydrate)) was purchased from Sigma (Cat# 71768); the DNA-FITC standard ((polyA (5'-(CH2)12-A19-3'-FITC) modified by 5'-amino and 3'-fluorescein)) was customized by Invitrogen Trading Shanghai Co., Ltd; and the buffer solution and the like for experiments were purchased from Sigma.

### 2. IC50 measurement of the PTP1 B by the compound 2

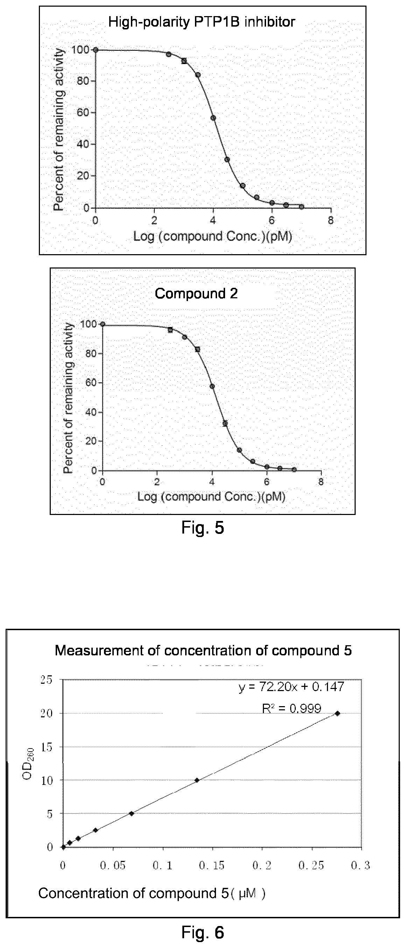

In order to trace influence of chemical modification on the activity of crude drugs, the activity of the compound 2 with respect to the PTP1 B was measured in the present invention. 10 µL of compound was added to 90 µL of reaction system containing substrate and PTP1 B, with a final concentration of 10 µM, 3 µM, 1 µM, 0.3 µM, 0.1 µM, 0.03 µM, 0.01 uM, 0.003 µM, 0.001 µM, 0.0003 µM and 0 µM, respectively; and the compound reacted for 15 min at room temperature, and the absorption value was measured at 405 nm every 60 s. The relative percentage of the reaction rate at each concentration point was calculated, assuming the reaction rate without any compound (the amount of increase of the absorption value/the reaction time) as 100%. Curve fitting was performed, by GraphPad Prism drawing software, in a sigmoidal dose-response (variable slope) model, and the IC50 value of the compound to be tested was calculated. (See Fig. 5 for IC50 curve of the compound 2)

### 3. Measurement of the content of the compound 5.

The DNA-FITC standard (100 µM) was diluted to 20 µM, 10 µM, 5 µM, 2.5 µM, 1.25 µM, 0.625 µM, and 0.3125 µM, the OD260absorption value was detected in a TECAN microplate reader, and a standard curve was made by taking the detected value as Y-axis and the concentrate of the standard as X-axis. The detected value of the sample was substituted into the standard curve to obtain the concentration (see Fig. 6).

### 4. IC50 measurement of the PTP1 B by the compound 5

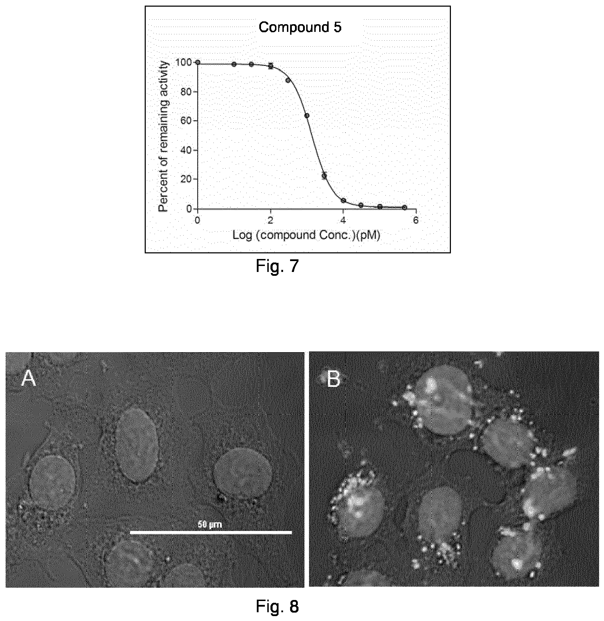

10 µL of compound was added to 90 µL of reaction system containing substrate and PTP1 B, with a final concentration of 10 µM, 3 µM, 1 µM, 0.3 µM, 0.1 µM, 0.03 µM, 0.01 µM, 0.003 µM, 0.001 µM, 0.0003 µM and 0 µM, respectively; and the compound reacted for 15 min at room temperature, and the absorption value was measured at 405 nm every 60 s. The relative percentage of the reaction rate at each concentration point was calculated, assuming the reaction rate without any compound (the amount of increase of the absorption value/the reaction time) as 100%. Curve fitting was performed, by GraphPad Prism drawing software, in a sigmoidal dose-response (variable slope) model, and the IC50 value of the compound to be tested was calculated (see Fig. 7).

In the experiment, the measurement of activity was performed with extracellular protease, and no membrane permeation effect of the compound was involved. Hence, it was unnecessary to consider the membrane permeability for all ingredients to be tested. It may be seen by comparing the above-mentioned experimental results that the modified compounds have similar in-vitro activity to that of the compounds before modification. This result shows that the compound drug-delivery precursor of the present invention will not reduce the bioactivity of the original compounds

### Embodiment 3

### Preparation of drug carriers based on compound drug-delivery precursor

### 1. Materials and reagents

The HepG2 cell strains were purchased from Shanghai Institutes for Bioscience Chinese Academy of Sciences; the RPMI-1640 culture medium was purchased from Hyclone; the fetal bovine serum was purchased from Tianjin Hao Yang Biological Products Co., Ltd.; the trypsin and Opti-MEM were purchased from Invitrogen; the X-tremeGENEsiRNA transfection reagent was purchased from Roche; and the cell culture dishes and other consumables were all purchased from Corning.

### 2. Preparation of drug carriers based on compound drug-delivery precursor

(1) Preparing materials: weighing the compound drug-delivery precursor and the drug carrier in a proportion of 1:10-100 (W/V); and
(2) Incubating: taking the preparation of a drug carrier for transferring a compound drug-delivery precursor (25 nM) as an example, 0.125 µg of compound drug-delivery precursor was added to a sterile centrifuge tube (tube A) of 1.5 mL, and mixed uniformly with Opti-MEM in a corresponding volume, with a total volume of 100 µL; the X-tremeGENEsiRNA reagent was shaken gently, and 2.5 µL of X-tremeGENEsiRNA reagent was mixed with 97.5 µL of Opti-MEM in another tube (tube B), with a total volume of 100 µL; and the solution in the tube A was mixed with the solution in the tube B, and the mixture was slightly triturated by a pipette and incubated for 20 min at room temperature. Then, the drug carrier based on the compound drug-delivery precursor (25 nM) was obtained.

### Embodiment 4

### Influence of the compound drug-delivery precursor on membrane transfer efficiency

### 1. Materials and reagents

TheHepG2 cell strains were purchased from Shanghai Institutes for Bioscience Chinese Academy of Sciences; the RPMI-1640 culture medium was purchased from Hyclone Shanghai; the fetal bovine serum was purchased from Tianjin Hao Yang Biological Products Co., Ltd.; the trypsin and Opti-MEM were purchased from Invitrogen Shanghai; the X-tremeGENEsiRNA transfection reagent was purchased from Roche China; the cell culture dishes and other consumables were all purchased from Corning China; and the compound 5 was provided by the Embodiment 1.

### 2. Preparation before transfer of single-stranded or double-stranded DNA/RNA in different sequences

24h before transfer, the HepG2 cells in the phase of logarithmic growth were digested with trypsin; a culture medium containing 10 % serum was used for adjusting the cell density to 1.0×10⁷ cells/20 mL; and the cells were inoculated again in a cell culture dish of 15 cm and cultured in a culture incubator containing 5% CO2at 37°C. The cells may be used for experiments when the cell density reaches 60% to 70% 24h later.

### 3. Transfer

4 nmol of compound 5 was added to a sterile centrifuge tube (tube A) of 15 mL, and mixed uniformly with Opti-MEM in a corresponding volume, with a total volume of 2 mL; the X-tremeGENEsiRNA reagent was shaken gently, and 160 µL of X-tremeGENEsiRNA reagent was mixed with 1.84 mL of Opti-MEM in another tube (tube B); and the solution in the tube A was mixed with the solution in the tube B, the mixture was slightly triturated by a pipette and incubated for 20 min at room temperature.

6 mL of RPMI-1640 serum-free culture medium was added to the mixture and mixed uniformly; the primary culture medium in the HepG2 cell culture dish was discarded, and slightly triturated with RPMI-1640 serum-free culture medium once; and then, the mixture was moved into the HepG2-PT cell culture dish, and cultured in a culture incubator containing 5% CO2at 37°C. 6h later, the positioning of the compound in the cells was observed by a laser confocal microscope.

### 4. Experimental results

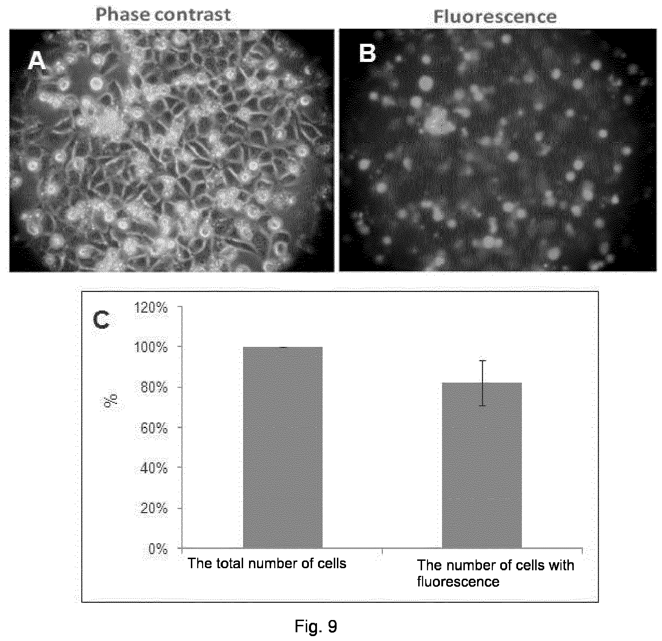

The results are as shown in Fig. 8 , an original compound 2 can not enter the cells after being linked to the fluorescent tag (see Fig. 8A); the drug-delivery precursor compound 5 may be transferred by the X-tremesiRNA into calls, with most of the compound 5 into the cytoplasm and a few of the compound 5 into the cell nucleus (see Fig. 8B); and the transfer efficiency may reach over 80%(see Fig. 9).

### Embodiment 5

### Change in cell transfer and phosphorylation level of the drug carrier based on the compound drug-delivery precursors

### 1. Materials and reagents

The HepG2 cell strains were purchased from Shanghai Institutes for Bioscience Chinese Academy of Sciences; the RPMI-1640 culture medium was purchased from Hyclone; the fetal bovine serum was purchased from Tianjin Hao Yang Biological Products Co., Ltd.; the trypsin was purchased from Invitrogen; the cell lysis buffer and the protease inhibitor were purchased from Pierce; the P-IRS-1 ELSA kit was purchased from Bio-swamp; and the cell culture dishes and other consumables were all purchased from Corning.

### 2. Study on influence, of the drug carrier based on the compound drug-delivery precursor, on the membrane permeation and transfer of the compound

Protein tyrosine phosphatase-1B (PTP1B), belonging to the family of protein tyrosine phosphatases (PTPs) and existing in two forms of transmembrane receptor-like protein and endoenzyme, catalyzes the dephosphorylation reaction of phosphorylated tyrosine residues of protein, and is the first PTPs^{[2.3]}identified and purified in mammalian bodies. PTP1 B acts on proteins related to insulin-signaling transduction, such as, insulin receptor (IR), insulin receptor substrates 1, 2 (IRS-1, IRS-2), growth factor receptor bound protein 2 (Grb2) and phosphatidylinositol 3 kinase (PI-3K), so that the phosphorylated tyrosine residues of these proteins are dephosphorylated, thereby attenuating the insulin-signaling transduction, thus producing post-receptor insulin resistance^{[4]}. Therefore, in the present invention, by measuring change in IRS-1 phosphorylation level in cells after drugs are transferred into the cells, the influence of drugs on the insulin-signaling pathway in the cells after the drugs enter the cells is evaluated. A verification method is as follows:
2.1 24h before transfer, the HepG2 cells in the phase of logarithmic growth were digested with trypsin; a culture medium containing 10 % serum was used for adjusting the cell density to 0.5×10⁶cells/mL; and the cells were inoculated again in a six-pore plate and cultured in a culture incubator containing 5% CO2for 24h at 37°C. The cells may be used for experiments when the cell density achieves 60% to 70% 24h later.
2.2 0.025 µg of compound drug-delivery precursor and 0.075 µg of compound drug-delivery precursor were added to two sterile centrifuge tubes (tubes A1, A2) of 1.5 mL, respectively, and uniformly mixed with Opti-MEM in a corresponding volume, with a total volume of 100 µL; the X-tremeGENEsiRNA reagent was shaken gently, and 2.5 µL of X-tremeGENEsiRNA reagent was mixed with 97.5 µL of Opti-MEM in other two tubes (tubes B1, B2), with a total volume of 100 µL; and the solution in the tube A was mixed with the solution in the tube B, slightly triturated by a pipette, and incubated for 20 min at room temperature. In this way, the drug carrier for the compound drug-delivery precursor was obtained. Operations similar to the above were repeated, with cases without compounds or without X-tremeGENEsiRNA or without both as two contrast controls and a blank control, respectively.
2.3 800 µL of RPMI-1640 serum-free culture medium was added to the mixture and mixed uniformly; the primary culture medium in the HepG2 cell culture dish was discarded, and slightly washed with RPMI-1640 serum-free culture medium once; then the blank control, the X-tremeGENEsiRNA transfection reagent, the compound drug-delivery precursor and the drug carrier for the compound drug-delivery precursor were moved into the HepG2 cell culture dish, and cultured in a culture incubator containing 5% CO2at 37°C for 5h. 1 µg/mL of insulin and glucose (5 mM) were added for induction for half an hour.
2.4 The cells were washed with ice-cold PBS for three times; 50 µL of cell lysis buffer was added in each pore for lysis on ice for 1 h; and then, the solution was centrifuged, the supernatant was collected, and protein quantization was performed by a BCA kit. A same amount of total protein was added into an ELISA plate, and the phosphorylation level of IRS-1 was measured by an ELISA kit. Four parallel pores were designed in each experiment, and the data came from three independent experiments.

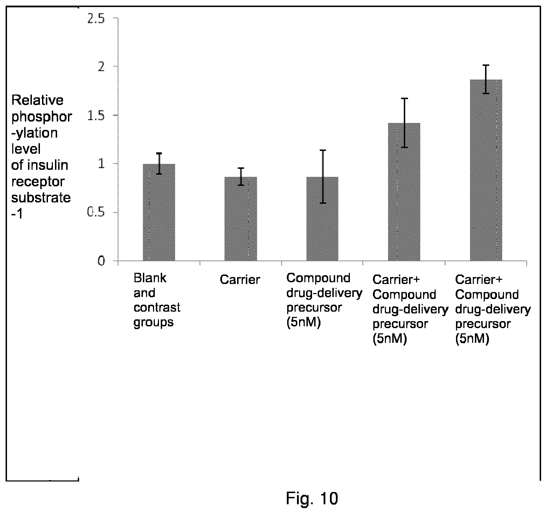

Experimental results are as shown in Fig. 10 .

It may be seen from Fig. 10 that, after the drugs having different concentration were transferred into HepG2cells, compared with the blank control without drugs, the phosphorylation of IRS-1 in the cells is increased. It is indicated that, by the compound drug-delivery precursor and drug carrier in the present invention, drugs with low membrane permeability are transferred into cells, and the drugs are ensured to play their functions in the cells.

### Embodiment 6

### Synthesis route of drug-delivery precursor 2 of the present invention

The synthesis route of the drug-delivery precursor 2 for transmembrane transfer is as follows:

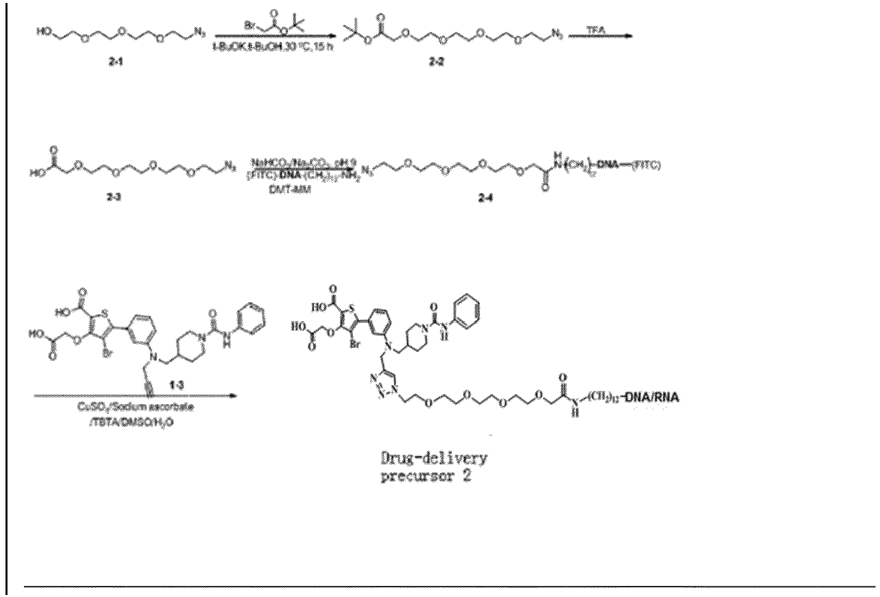

### Compound 2-2: 14-azido-3,6,9,12-tetraoxatetradecyl-1-tert-butyl carboxyl

Potassium tert-butylate (336 mg, 3 mmol) was added to the compound (2-1) (372 mg, 2 mmol) in 15 mL of tert-butanol solution, and stirred for 15 min at 30°C. Then, tert-butyl bromoacetate (780 mg, 4 mmol) was added to the system, and stirred overnight at 30°C. The system is distilled under reduced pressure to obtain a crude product. The crude product was dissolved into 30 mL of dichloromethane, and washed with water for three times and with saturated salt water for three times successively; and the organic phase was dried by anhydrous sodium sulfate, filtered and concentrated to obtain a compound (2-2) (clear oily liquid, 466 mg, with a yield of 70%). MS m/z (ESI): 250(M-tBu-N2+H)⁺; 278(M-tBu+H)⁺

### Compound 2-3: 14-azido-3,6,9,12-tetraoxatetradecyl-1-carboxyl

Trifluoroacetic acid (1 mL) was added to the compound (2-2) (466 mg, 1.4 mmol) in 5 mL of dichloromethane solution, and stirred for 2h at room temperature. The solution is concentrated to obtain a crude product compound (2-3) (clear oily liquid, 370 mg, with a yield of 95%). MS m/z (ESI): 250(M-N2+H)⁺; 278(M+H)⁺

### Compound 2-4: 14-azido-3,6,9,12-tetraoxatetradecyl-1-formyl-n-dodecyl 19 polyA fluorescein

A mixture of polyA (5'-(CH2)12-A19-3'-FITC) (80 nmol) modified by 5'-amino and 3'-fluorescein, the compound (2-3) (1.6 µmol, 200 eq.), 4-(4,6-dimethoxy triazin-2yl)-4-methyl morpholine hydrochloride (DMT-MM, 1.6 µmol,200 eq.) in 80 µL of 0.5 M sodium carbonate/sodium bicarbonate buffer solution (pH 9), 160 µL of deionized water and 160 µL of dimethylsulfoxide was shaken overnight in a low speed at room temperature. Then, the reaction system was directly separated by reverse HPLC column chromatography and lyophilized to obtain the compound (2-4) (white solid). MS m/z (TOF): 6896

### Drug-delivery precursor 2:

60 µL of solution A (copper sulfate and tris[(1-benzyl-1 H-1,2,3-triazol-4-yl)methyl]amine were dissolved at a mole ratio of 1:2 into a solution composed of water, dimethylsulfoxide and tert-butanol at a volume ratio of 4:3:1, with a concentration of 10 mM) was added to solution B (compound (2-4) (50 nmol) in 400 µL of aqueous solution and 4-bromo-3-oxoacetic acid-5-(3-(((1-phenyl carbamoylpiperidine)-4-methyl)-N-propargyl amine) phenyl) thiophene-2-formic acid (compound 1-3) (3 µmol)) in 100 µL of DMSO solution, and vortex-centrifuged; and subsequently, 120 µL of newly-prepared sodium ascorbate (1200 nmol) in aqueous solution was added to the reaction system, and shaken overnight in a low speed at room temperature. Then, the reaction solution was directly separated by reverse HPLC column chromatography and purified to obtain the product (drug-delivery precursor 2) (light yellow solid). MS m/z (TOF): 7521

### Embodiment 7

### Synthesis route of drug-delivery precursor 3 of the present invention

The synthesis route of the drug-delivery precursor 3 for transmembrane transfer is as follows:

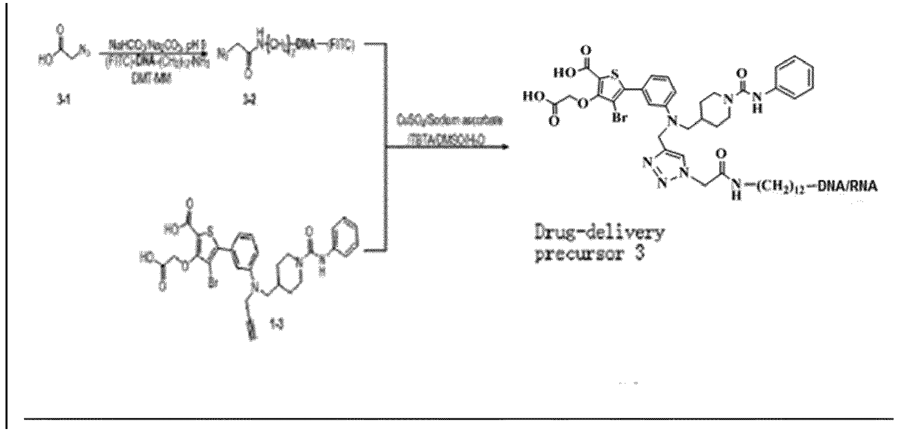

### Compound 3-2:

A mixture of polyA (5'-(CH2)12-A19-3'-FITC) (80 nmol) modified by 5'-amino and 3'-fluorescein, azidoacetic acid (3-1) (1.6 µmol, 200 eq.), 4-(4,6-dimethoxy triazin-2-yl)-4-methyl morpholine hydrochloride (DMT-MM, 1.6 µmοl, 200 eq.) in 80 µL of 0.5 M sodium carbonate/sodium bicarbonate buffer solution (pH 9), 160 µL of deionized water and 160 µL of dimethylsulfoxide was shaken overnight in a low speed at room temperature. Then, the reaction system was directly separated by reverse HPLC column chromatography and lyophilized to obtain the compound (4) (white solid). MS m/z (TOF): 6720

### Drug-delivery precursor 3:

60 µL of solution A (copper sulfate and tris[(1-benzyl-1 H-1,2,3-triazol-4-yl)methyl]amine were dissolved at a mole ratio of 1:2 into a solution composed of water, dimethylsulfoxide and tert-butanol at a volume ratio of 4:3:1, with a concentration of 10 mM) was added to solution B (compound (3-2) (50 nmol) in 400 µL of aqueous solution and 4-bromo-3-oxoacetic acid-5-(3-(((1-phenyl carbamoylpiperidine)-4-methyl)-N-propargyl amine) phenyl) thiophene-2-formic acid (1-3) (3 µmol)) in 100 µL of DMSO solution, and vortex-centrifuged; and subsequently, 120 µL of newly-prepared sodium ascorbate (1200 nmol) in aqueous solution was added to the reaction system, and then shaken overnight in a low speed at room temperature. Then, the reaction solution was directly separated by reverse HPLC column chromatography and purified to obtain the product (drug-delivery precursor 3) (light yellow solid). MS m/z (TOF): 7345

### Embodiment 8

### Synthesis route of drug-delivery precursor 4 of the present invention

The synthesis route of the drug-delivery precursor 4 for transmembrane transfer is as follows:

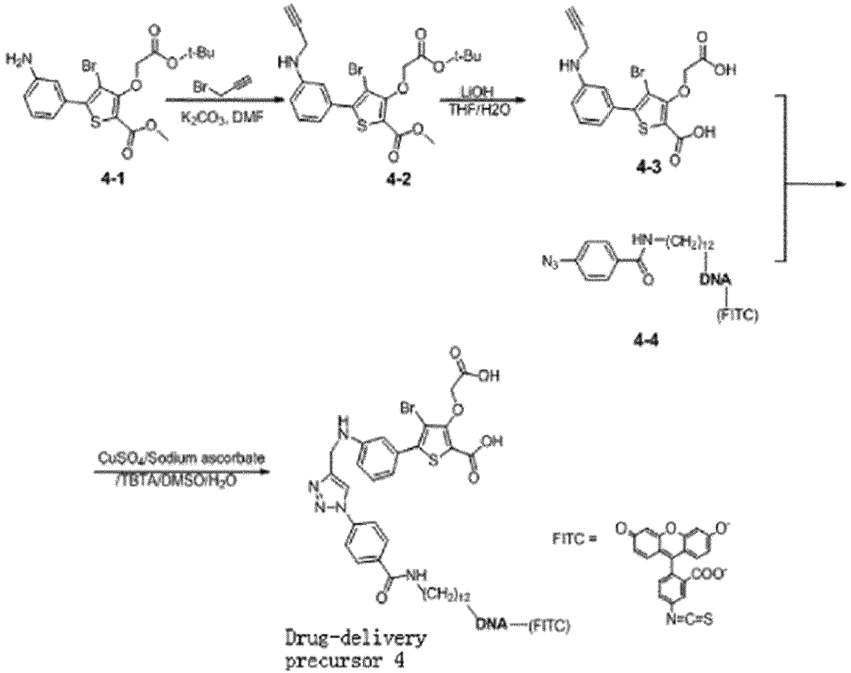

### Synthesis of compound 4-2

The compound (4-1) (441 mg, 1 mmol), propargyl bromide (95 mg, 0.8 mmol) and potassium carbonate (138 mg, 1 mmol) were dissolved into 20 mL of N,N-dimethylformamide, and stirred overnight at room temperature. The system is distilled under reduced pressure to obtain a crude product. The crude product was dissolved into 50 mL of dichloromethane, and washed with water for three times and with saturated salt water for three times successively; the organic phase was dried by anhydrous sodium sulfate, filtered and concentrated to obtain a compound (4-2) (yellow solid, 287 mg, with a yield of 60%). MS m/z (ESI): 424(M-tBu+H)⁺; 480 (M+H)⁺.

### Synthesis of compound 4-3

The compound (4-2) (87 mg, 0.6 mmol) and lithium hydroxide monohydrate (126 mg, 3 mmol) were dissolved into 5 mL of methanol and 5 mL of water, and reflux-stirred overnight. Ethanol was removed by distillation. The solution was diluted with 20 mL of water and acidified with 1 N HCl until the pH became 2.0, and lyophilized to obtain a crude product; and the crude product was directly subject to reverse high-phase liquid-phase separation to obtain the compound (4-3) (yellow solid, 216 mg, with a yield of 80%). MS m/z (ESI): 410(M+H)⁺.

### Synthesis of drug-delivery precursor 4

60 µL solution A (copper sulfate and tris[(1-benzyl-1 H-1,2,3-triazol-4-yl)methyl]amine were dissolved at a mole ratio of 1:2 into a solution composed of water, dimethylsulfoxide and tert-butanol at a volume ratio of 4:3:1, with a concentration of 10 mM) was added to solution B (the compound (4-4) (50 nmol) in 400 µL of aqueous solution and the compound (4-3) (3 µmol) in 100 µL of DMSO solution), and vortex-centrifuged; and subsequently, 120 µL of newly-prepared sodium ascorbate (1200 nmol) in aqueous solution was added to the reaction system, and then shaken overnight in a low speed at room temperature. Then, the reaction solution was directly separated by reverse HPLC column chromatography and purified to obtain the product (drug-delivery precursor 4) (light yellow solid). MS m/z (TOF): 7191

### Embodiment 9

### Evaluation on transmembrane transfer efficiency of drug-delivery precursors having single-stranded or double-stranded DNA or RNA of different length, different compounds and different linkers

### 1. Materials and reagents

The HepG2 cell strains were purchased from Shanghai Institutes for Bioscience Chinese Academy of Sciences; the RPMI-1640 culture medium was purchased from Hyclone Shanghai; the fetal bovine serum was purchased from Tianjin Hao Yang Biological Products Co., Ltd.; the trypsin and Opti-MEM were purchased from Invitrogen Shanghai; the X-tremeGENEsiRNA transfection reagent was purchased from Roche China; the cell culture dishes and other consumables were all purchased from Corning China; polyA 5'-NH2-(CH2)12-PO4-A5-3'-FITC of 5 bp, polyA 5'-NH2-(CH2)12-PO4-A19-3'-FITC of 19 bp, polyA 5'-NH2-(CH2)12-PO4-A38-3'-FITC of 38 bp, Single-stranded random sequence 5'-NH2-(CH2)12-PO4-TGGGCTGGCCAAACTGCTG-3'-FITC of 19 bp, and double-stranded random sequence (5'-NH2-(CH2)12-PO4-TGGGCTGGCCAAACTGCTG-3'-FITC: 5'-CAGCAGTTTGGCCAGCCCA-3') of 19 bp were all synthesized by Invitrogen Trading Shanghai; and the drug-delivery precursors 2 to 4 are prepared by the embodiments 6 to 8, respectively.

### 2. Preparation before transfer

24h before transfer, the HepG2 cells in the phase of logarithmic growth were digested with trypsin; a culture medium containing 10 % serum was used for adjusting the cell density to 0.5×10⁶cells/mL; and the cells were inoculated again in a cell culture dish of 15 cm and cultured in a culture incubator containing 5% CO2at 37°C. The cells may be used for experiments when the cell density reaches 60% to 70% 24h later.

### 3. Transfer

4 nmol of the above-mentioned single-stranded or double-stranded DNA/RNA fragments in different sequences and various drug-delivery precursors was added to a sterile centrifuge tube (tube A) of 15 mL, respectively, and uniformly mixed with Opti-MEM in a corresponding volume, with a total volume of 2 mL; the X-tremeGENEsiRNA reagent was shaken gently, and 160 µL of X-tremeGENEsiRNA reagent was mixed with 1.84 mL of Opti-MEM in another tube (tube B); and the solution in the tube A was mixed with the solution in the tube B, the mixture was slightly triturated by a pipette and incubated for 20 min at room temperature.

6 mL of RPMI-1640 serum-free culture medium was added to the mixture and mixed uniformly; the primary culture medium in the HepG2 cell culture dish was discarded, and slightly triturated with RPMI-1640 serum-free culture medium once; and then, the mixture was moved into the HepG2-PT cell culture dish, and cultured in a culture incubator containing 5% CO2at 37°C. 6h later, the positioning of the compound in the cells was observed by a laser confocal microscope.

### 4. Experimental results

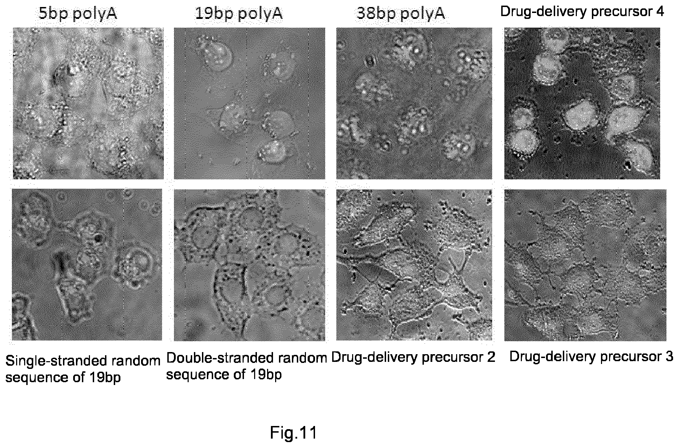

The results are as shown in Fig. 11 , single-stranded or double-stranded DNA or RNA random or polyA fragments of not less than 5 bp (for example, polyA of 5 bp, polyA of 19 bp, polyA of 38 bp, single-stranded random sequence fragments of 19 bp and double-stranded random sequence fragments of 19 bp) and various drug-delivery precursors of the present invention may be all transferred into cells by X-tremesiRNA, with most of them into the cytoplasm and a few of them into the cell nucleus.

In conclusion, the drug-delivery precursor and drug carrier preparation of the present invention may effectively improve the membrane permeability of compounds with low membrane permeability and provide a new choice for clinical medication.

### Reference documents

[1] Klopman G, Zhu H. Recent methodologies for the estimation of n-octanol/water partition coefficients and their use in the prediction of membrane transport properties of drugs [J]. MiniRev Med Chem, 2005, 5(2):127-133
[2] A.R. Saltiel, New perspectives into the molecular pathogenesis and treatment of type2 diabetes[J], Cell, 2001; 104: 517-529 .
[3] Z.Y. Zhang, Protein tyrosine phosphatases: structure and function, substrate specificity, and inhibitor development[J].Annu. Rev. Pharmacol.Toxil. 2002; 42: 209-234 .
[4] H. Charbonneau, N.K. Tonks, S. Kumar, et al. Human placenta protein tyrosine phosphatase: amino acid sequence and relationship to a family of receptor-like proteins[J]. Proc. Natl. Acad. Sci. USA. 1989; 86: 5252-5256 .

## Claims

1. A compound drug-delivery precursor, **characterized in that** the structural formula of the compound drug-delivery precursor is as follows:
X-linker-DNA/RNA Formula 1
where X has difficulties to pass a membrane, and linker is a linking group between X and DNA or RNA allowing any covalent linkage enabling the compatible linkage and reaction between the X and the DNA or RNA; wherein the compound drug-delivery precursor has the structural formula of

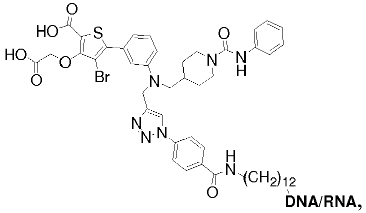

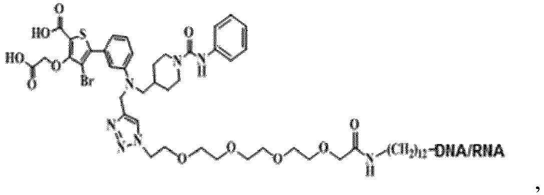

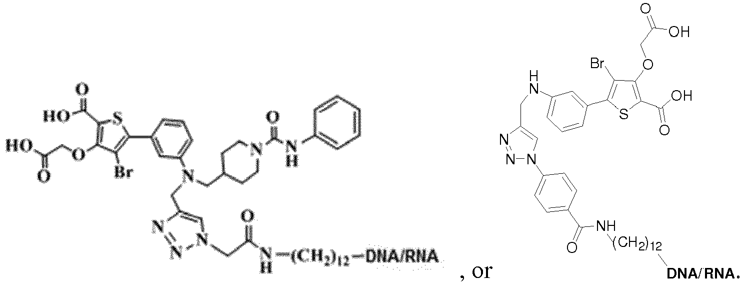

2. The compound drug-delivery precursor according to claim 1, **characterized in that** the DNA or RNA is single-stranded or double-stranded.

3. The compound drug-delivery precursor according to claim 2, **characterized in that** the length of the single-strand or double-strand of the DNA or RNA ranges from 5 to 38 base pairs or bases.

4. The compound drug-delivery precursor according to claim 3, **characterized in that** the DNA or RNA is any sequence within a defined range of length.

5. The compound drug-delivery precursor according to claim 1, **characterized in that** the DNA or RNA has a functional group for covalent linkage.

6. The compound drug-delivery precursor according to any one of claims 1 to 5, **characterized in that** the DNA or RNA is tagged with biotin, fluorescein or isotope for tracing.

7. The compound drug-delivery precursor according to claim 1, **characterized in that** a linking site of the linker on the compound X has no influence on the bioactivity of the X.

8. The compound drug-delivery precursor according to claim 1, **characterized in that** the linker is covalently linked to the compound X directly or by pre-modified compound X.

9. The method for preparing the compound drug-delivery precursor according to claim 1, comprising the following operating steps of:
1) Preparing raw materials: preparing compound X and DNA or RNA with one reactive functional group;
(2) Preparing for covalent linkage of the compound X: selectively reacting the compound X with one functional group of a bifunctional reagent, to obtain a compound X1;
(3) Preparing for covalent linkage of the DNA or RNA: modifying the DNA or RNA with one reactive functional group with the bifunctional reagent, to obtain modified DNA or RNA matched with the covalent linkage of the compound X in the step (2); and
(4) Covalently linking: covalently linking the compound X1 to the modified DNA or RNA matched with the compound X1, to obtain the compound drug-delivery precursor by separation and purification; or covalently linking the compound X1 to the DNA or RNA with one reactive functional group in the step (1), to obtain the compound drug-delivery precursor by separation and purification; or covalently linking the compound X to the modified DNA or RNA in the step (3), to obtain the compound drug-delivery precursor by separation and purification.

10. A drug carrier preparation based on a compound drug-delivery precursor, **characterized in that** the drug carrier preparation is prepared from the compound drug-delivery precursor according to any one of claims 1 to 8 and a carrier.

11. The drug carrier preparation based on a compound drug-delivery precursor according to claim 13, **characterized in that** the carrier is a biological material for transferring DNA or RNA.

12. A method for preparing the drug carrier preparation based on a compound drug-delivery precursor according to claim 13, comprising the following operating steps of:
(1) Preparing materials: weighing the compound drug-delivery precursor and the drug carrier in a proportion of 1:10-100 (W/V); and
(2) incubating: uniformly mixing the compound drug-delivery precursor with a buffer solution to obtain a solution A; adding the drug carrier to the buffer solution and uniformly mixing to obtain a solution B; and mixing the solution A and the solution B, triturating by a pipette, and incubating at room temperature to obtain the drug carrier preparation; optionally wherein in the step (2), the incubation at room temperature lasts for 20 min.

## Patentansprüche

1. Applikationsvorläufer einer Verbindung, **dadurch gekennzeichnet, dass** die Strukturformel des Applikationsvorläufers einer Verbindung wie folgt ist:
X-linker-DNA/RNA Formel 1
wobei X schwer durch die Zellmembran durchdringen kann; und wobei linker die Verknüpfungsgruppe zwischen X und DNA oder RNA darstellt und zum Realisieren einer Kompatibilitätsbindung und irgendeines kovalenten Bindung der Reaktion zwischen X und DNA oder RNA dient; und wobei die Strukturformel des Applikationsvorläufers einer Verbindung wie folgt ist:

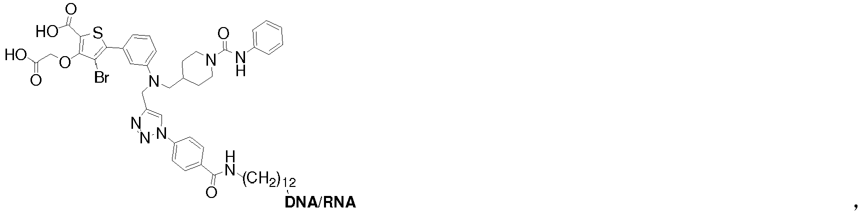

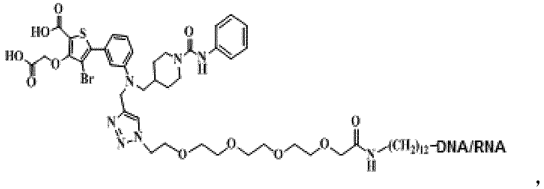

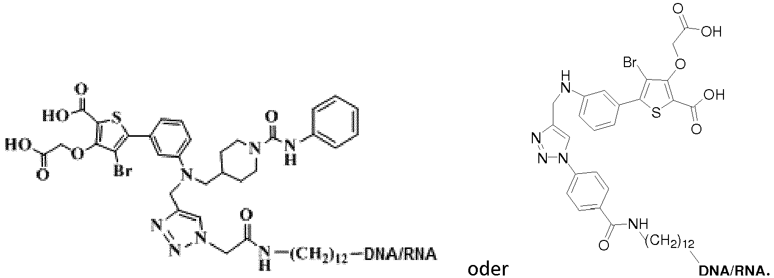

2. Applikationsvorläufer einer Verbindung nach Anspruch 1, **dadurch gekennzeichnet, dass** die DNA oder RNA einzelsträngig oder doppelsträngig ist.

3. Applikationsvorläufer einer Verbindung nach Anspruch 2, **dadurch gekennzeichnet, dass** der Einzelstrang oder Doppelstrang von der DNA oder RNA eine Länge von 5 bis 38 Basenpaaren oder Basen aufweist.

4. Applikationsvorläufer einer Verbindung nach Anspruch 3, **dadurch gekennzeichnet, dass** bei der DNA oder RNA es sich um eine beliebige Sequenz innerhalb eines definierten Längenbereichs handelt.

5. Applikationsvorläufer einer Verbindung nach Anspruch 1, **dadurch gekennzeichnet, dass** die DNA oder RNA eine funktionelle Gruppe zur kovalenten Bindung aufweist.

6. Applikationsvorläufer einer Verbindung nach Anspruch 1, **dadurch gekennzeichnet, dass** die DNA oder RNA mit Biotin, Fluoreszenz oder Isotop zur Verfolgung markiert ist.

7. Applikationsvorläufer einer Verbindung nach Anspruch 1, **dadurch gekennzeichnet, dass** die Bindungsstelle von linker an der Verbindung X keinen Einfluss auf die biologische Aktivität von X hat.

8. Applikationsvorläufer einer Verbindung nach Anspruch 1, **dadurch gekennzeichnet, dass** linker und die Verbindung X eine direkte kovalente Bindung herstellen oder durch eine Pre-modifizierte Verbindung X miteinander gebunden werden.

9. Herstellungsverfahren des Applikationsvorläufers einer Verbindung nach Anspruch 1, **dadurch gekennzeichnet, dass** es folgende Schritte umfasst:
(1) Holen der Rohmaterialien: Holen einer Verbindung X und einer DNA oder RNA mit einer reaktiven funktionellen Gruppe;
(2) Vorbereitung für die kovalente Bildung der Verbindung X: die Verbindung X reagiert selektiv mit einer funktionellen Gruppe eines Reagenz mit doppelten funktionellen Gruppen, um eine Verbindung X1 zu erhalten;
(3) Vorbereitung für die kovalente Bindung von DNA oder RNA: die DNA oder RNA mit einer reaktiven funktionellen Gruppe wird mit einem Reagenz mit doppelten funktionellen Gruppen modifiziert, um eine modifizierte DNA oder RNA zu erhalten, welches sich an die kovalente Bindung der Verbindung X in dem Schritt (2) anpasst;
(4) Kovalente Bindung: eine kovalente Bindung wird für die Verbindung X1 und eine daran angepasste modifizierte DNA oder RNA hergestellt, nach Ausscheiden und Reinigen wird ein Applikationsvorläufer einer Verbindung erhalten; oder eine kovalente Bindung wird für die Verbindung X1 und eine DNA oder RNA mit einer reaktiven funktionellen Gruppe hergestellt, nach Ausscheiden und Reinigen wird ein Applikationsvorläufer einer Verbindung erhalten; oder eine kovalente Bindung wird für die Verbindung X und die modifizierte DNA oder RNA in dem Schritt (3) hergestellt, nach Ausscheiden und Reinigen wird ein Applikationsvorläufer einer Verbindung erhalten.

10. Auf dem Applikationsvorläufer einer Verbindung basierendes Arzneimittelträgerpräparat, **dadurch gekennzeichnet, dass** es durch einen Applikationsvorläufer einer Verbindung nach einem der Ansprüche 1 bis 8 und einen Träger hergestellt wird.

11. Auf dem Applikationsvorläufer einer Verbindung basierendes Arzneimittelträgerpräparat nach Anspruch 10, **dadurch gekennzeichnet, dass** der Träger Biomaterialien für die DNA oder RNA transportiert.

12. Herstellungsverfahren für ein auf dem Applikationsvorläufer einer Verbindung basierendes Arzneimittelträgerpräparat nach Anspruch 10, **dadurch gekennzeichnet, dass** es folgende Schritte umfasst:
(1) Holen der Rohmaterialien: Holen eines Applikationsvorläufers einer Verbindung und eines Arzneimittelträgers mit einem Verhältnis von 1:10-100 (W/V);
(2) Inkubation: Holen eines Applikationsvorläufers einer Verbindung, gleichmäßiges Mischen mit einer Pufferlösung, um die Lösung A zu erhalten; Holen eines Arzneimittelträgers, Zugeben der Pufferlösung, nach gleichmäßigem Mischen wird die Lösung B erhalten; Mischen der Lösung A und der Lösung B, Blasen mit Pistolenkopf, Inkubation bei Raumtemperatur, dann wird ein Arzneimittelträgerpräparat erhalten; wobei in dem Schritt (2) die Inkubationszeit bei Raumtemperatur 20 min beträgt.

## Revendications

1. Précurseur d'administration de médicament composé, **caractérisé en ce que** Ia formule structurelle du précurseur d'administration de médicament composé est Ia suivante:
X-liant-ADN/ARN Formule 1
où X a des difficultés à passer une membrane, et un liant est un groupe de liaison entre X et l'ADN ou l'ARN permettant n'importe quelle liaison covalente permettant Ia liaison et Ia réaction compatibles entre le X et l'ADN ou l'ARN; dans lequel le précurseur d'administration de médicament composé a Ia formule structurelle suivante:

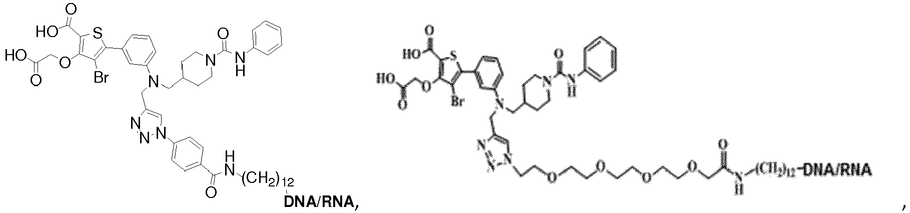

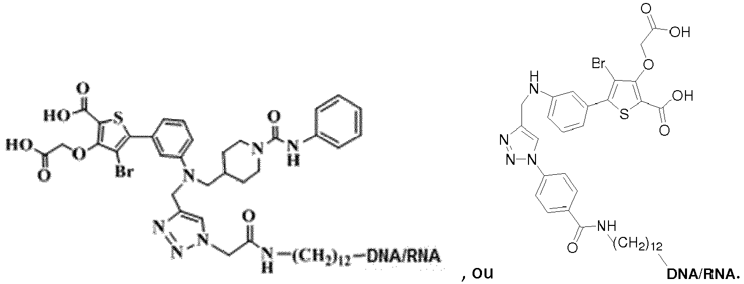

2. Précurseur d'administration de médicament composé selon Ia revendication 1, **caractérisé en ce que** l'ADN ou l'ARN est monocaténaire ou bicaténaire.

3. Précurseur d'administarion de médicament composé selon Ia revendication 2, **caractérisé en ce que** Ia longueur du brin simple ou double brin de l'ADN ou de l'ARN est comprise entre 5 à 38 paires de bases ou bases.

4. Précurseur d'administration de médicament composé selon Ia revendication 3, **caractérisé en ce que** l'ADN ou l'ARN est toute séquence dans une plage de longueur définie.

5. Précurseur d'administration de médicament composé selon Ia revendication 1, **caractérisé en ce que** l'ADN ou l'ARN a un groupe fonctionnel pour la liaison covalente.

6. Précurseur d'administration de médicament composé selon l'une quelconque des revendications 1 à 5, **caractérisé en ce que** l'ADN ou l'ARN est marqué avec de la biotine, un isotope fluorescéinor pour le traçage.

7. Précurseur d'administration de médicament composé selon Ia revendication 1, **caractérisé en ce qu'**un site de liaison du lieur sur le composé X n'a aucune influence sur Ia bioactivité du X.

8. Précurseur d'administration de médicament composé selon la revendication 1, **caractérisé en ce que** le liant est lié de manière covalente au composé X directement ou par le composé X préalablement modifié.

9. Procédé de préparation du précurseur d'administarion de médicament composé selon Ia revendication 1, comprenant les étapes de fonctionnement suivantes:
1) Préparation des matières premières: préparation du composé X et de l'ADN ou de l'ARN avec un groupe fonctionnel réactif;
(2) Préparation de Ia liaison covalente du composé X: Ia réaction sélective du composé X avec un groupe fonctionnel d'un réactif bifonctionnel, pour obtenir un composé X1;
(3) Préparation pour Ia liaison covalente de l'ADN ou de l'ARN: Ia modification de l'ADN ou de l'ARN avec un groupe fonctionnel réactif avec le réactif bifonctionnel, pour obtenir un ADN ou un ARN modifié adapté à Ia liaison covalente du composé X dans l'étape (2); et
(4) Liaison covalente: lier par covalence le composé X1 à l'ADN ou à l'ARN modifié apparié avec le composé X1, pour obtenir le précurseur d'administration de médicament composé par séparation et purification; ou lier par covalence le composé X1 à l'ADN ou à l'ARN avec un groupe fonctionnel réactif dans l'étape (1), pour obtenir le précurseur d'administration de médicament composé par séparation et purification; ou lier par covalence le composé X à l'ADN ou à l'ARN modifié dans l'étape (3), pour obtenir le précurseur d'administration de médicament composé par séparation et purification.

10. Préparation de vecteur de médicament basée sur un précurseur d'administration de médicament composé, **caractérisée en ce que** Ia préparation de vecteur de médicament est préparée à partir du précurseur d'administration de médicament composé selon l'une quelconque des revendications 1 à 8 et d'un vecteur.

11. Préparation de vecteur de médicament à base d'un précurseur d'administration de médicament composé selon Ia revendication 10, **caractérisée en ce que** le vecteur est un matériel biologique pour le transfert d'ADN ou d'ARN.

12. Procédé de préparation de Ia préparation de vecteur de médicament à base d'un précurseur d'administration de médicament composé selon Ia revendication 10, comprenant les étapes de fonctionnement suivantes:
(1) Préparation des matériaux: peser le précurseur de médicament-administration composé et le vecteur de médicament dans une proportion de 1: 10-100 (W/V); et
(2) incubation: mélanger uniformément le précurseur d'administration de médicament composé avec une solution tampon pour obtenir une solution A; ajouter le vecteur de médicament à Ia solution tampon et mélanger uniformément pour obtenir une solution B; et mélanger la solution A et Ia solution B, triturer à l'aide d'une pipette, et incuber à température ambiante pour obtenir Ia préparation de vecteur de médicament; éventuellement dans lequel dans l'étape (2), l'incubation à température ambiante dure 20 min.


### SEQUENCE LISTING

<110> HitGen LTD.
<120> DRUG TARGET CAPTURING METHOD
<130> WK15-081-SCXDYY-US-081
<160> 6
<170> PatentIn version 3.5
<210> 1
   <211> 5
   <212> DNA
   <213> Artificial Sequence
<220>
   <223> 5bp polyA
<400> 1
   aaaaa 5
<210> 2
   <211> 19
   <212> DNA
   <213> Artificial Sequence
<220>
   <223> 19 bp polyA
<400> 2
   aaaaaaaaaa aaaaaaaaa 19
<210> 3
   <211> 38
   <212> DNA
   <213> Artificial Sequence
<220>
   <223> 38 bp ployA
<400> 3
   aaaaaaaaaa aaaaaaaaaa aaaaaaaaaa aaaaaaaa 38
<210> 4
   <211> 19
   <212> DNA
   <213> Artificial Sequence
<220>
   <223> 19bp single randon chain
<400> 4
   tgggctggcc aaactgctg 19
<210> 5
   <211> 19
   <212> DNA
   <213> Artificial Sequence
<220>
   <223> 19 bp Forward sequence of double-stranded random sequence
<400> 5
   tgggctggcc aaactgctg 19
<210> 6
   <211> 19
   <212> DNA
   <213> Artificial Sequence
<220>
   <223> 19 bp Reverse sequence of double-stranded random sequence
<400> 6
   cagcagtttg gccagccca 19